# EUROPÄISCHE PATENTSCHRIFT

(11) **EP 0 477 322 B1**
(45) Veröffentlichungstag und Bekanntmachung des Hinweises auf die Patenterteilung: **21.06.1995**
(21) Anmeldenummer: 91907144.9
(22) Anmeldetag: 12.04.1991
(51) Int. Cl.: G01N 33/52, G01N 33/543

(54) **TESTTRÄGER FÜR DIE ANALYSE VON FLÜSSIGKEITEN**
SLIDES FOR USE IN THE ANALYSIS OF LIQUIDS
SUPPORT DE TEST POUR L'ANALYSE DE LIQUIDES

(30) Priorität: 14.04.1990 DE 4012216
(43) Veröffentlichungstag der Anmeldung: 01.04.1992
(73) Patentinhaber: BOEHRINGER MANNHEIM GMBH, 68298 Mannheim (DE)
(72) Erfinder: KRAUSE, Manfred, D-6806 Viernheim (DE); SCHINDLER, Gerhard, D-6718 Grünstadt (DE)
(74) Vertreter: Pfeifer, Hans-Peter, Dr., Dr. H.-P. Pfeifer Dr. P. Jany, Patentanwälte
(86) Internationale Anmeldenummer: DE9100310
(87) Internationale Veröffentlichungsnummer: WO9116626

(56) Entgegenhaltungen:
- EP-A- 0 269 876
- EP-A- 0 334 015

## Beschreibung

Die Erfindung betrifft einen Testträger für die Analyse von Flüssigkeiten mit einem in einem Rahmen gehaltenen Testfeld, wobei der Rahmen ein Oberteil und ein Unterteil aufweist und mindestens das Oberteil eine Testfeldöffnung aufweist, durch die eine Probenflüssigkeit auf die Oberseite des Testfeldes aufgebracht werden kann.

Zur qualitativen oder quantitativen analytischen Bestimmung von Bestandteilen von Flüssigkeiten, insbesondere Körperflüssigkeiten von Menschen oder Tieren, werden zunehmend sogenannte trägergebundene Tests verwendet. Bei diesen sind Reagenzien in den Testschichten eines insgesamt als Testträger bezeichneten Analyseelementes eingebettet. Sie werden mit der Probe in Kontakt gebracht. Die Reaktion von Probe und Reagenzien führt zu einem nachweisbaren Signal, insbesondere einer Farbänderung, welche visuell oder mit Hilfe eines Gerätes, meistens reflexionsphotometrisch, ausgewertet werden kann. Es sind zahlreiche verschiedene Testträgertypen bekannt, die sich nicht nur bezüglich der verwendeten Reagenzien, sondern auch durch ihren Aufbau, insbesondere hinsichtlich der Anordnung und Befestigung der Testschichten, unterscheiden. Von besonderer praktischer Bedeutung sind folgende zwei Typen.

Streifenförmige Testträger bestehen im wesentlichen aus einer länglichen Tragschicht aus Kunststoffmaterial und darauf angebrachten Testschichten. Die Verbindung zwischen den Testschichten und dem Kunststoffträger wird meist durch Kleben hergestellt, wobei die Verklebung vielfach nicht auf der ganzen Fläche, sondern nur an einer Kante der Testschicht erfolgt oder zusätzliche Befestigungsmittel (Netze oder Befestigungsfolien) an der Tragschicht befestigt sind, welche indirekt die Testschichten festhalten. Dadurch ist eine sehr vielfältige Anordnung der Testschichten nebeneinander und/oder übereinander je nach den Anforderungen des einzelnen Tests möglich. Die Testschichten können unabhängig voneinander hergestellt und bei der Endmontage des streifenförmigen Testträgers zusammengefügt werden.

Die Erfindung richtet sich auf den eingangs erwähnten Testträgertyp, bei dem ein Testfeld ähnlich wie ein fotografisches Diapositiv von einem Rahmen gehalten wird. Sie werden im folgenden als "Testträger mit Rahmen" und in der englischsprachigen Literatur als "analysis slides" bezeichnet. Die Probe wird dabei üblicherweise durch die Testfeldöffnung auf eine Seite (Oberseite) des Testfeldes aufgebracht. Nach Ablauf der Testreaktion kann die Farbbildung - meist auf der von der Probenseite abgewandten Seite des Testfeldes (Unterseite)- beobachtet bzw. vermessen werden.

Ein Analyseelement mit einem aus einem Ober- und Unterteil bestehenden Rahmen und mehreren aufeinanderliegenden Testschichten ist beispielsweise aus der EP-A-0 334 015 bekannt.

Der Erfindung liegt die Aufgabe zugrunde, einen Testträger mit Rahmen zur Verfügung zu stellen, der sich durch vereinfachte Handhabung und verbesserte Analysesicherheit auszeichnet.

Die Aufgabe wird bei einem Testträger der eingangs bezeichneten Art dadurch gelöst, daß das Unterteil eine Aufnahme für das Testfeld aufweist, die Aufnahme in dem Unterteil von Positioniermitteln für das Testfeld umgeben ist, die Testfeldöffnung größer als das Testfeld ist, so daß der Rand des Testfeldes sichtbar ist, und das Oberteil in die Testfeldöffnung hineinragende, auf der Oberseite des Testfeldes aufliegende Andruckarme aufweist.

Bei den vorbekannten Testträgern mit Rahmen war die Testfeldöffnung durch welche die Blutprobe aufgetragen wird (Blutauftragsöffnung) stets kleiner als das Testfeld. Das Oberteil des Rahmens lag mit einer umlaufenden Andruckfläche am Rand des Testfeldes auf. Dies wurde für erforderlich gehalten, um eine ausreichende Positionierung und Planlage des Testfeldes zu erreichen. Außerdem wurde davon ausgegangen, daß nur mit einer Testfeldöffnung, die den Rand des Testfeldes abdeckt, ein seitliches Vorbeifließen des Blutes verhindert werden kann.

Hier zeigt die Erfindung einen grundlegend anderen Weg auf, der sich als außerordentlich vorteilhaft erweist.

Die Oberseite des Testfeldes ist praktisch vollständig zu sehen. Dadurch wird es möglich, visuell zu kontrollieren, ob eine ausreichende Menge Flüssigkeitsprobe aufgetragen wurde und das Testfeld vollständig benetzt ist. Insbesondere bei der Analyse von Blut läßt sich eine unvollständige Benetzung gut daran erkennen, daß Teile des flüssigkeitaufsaugenden Testfeldes nicht oder ungleichmäßig gefärbt werden. Die Handhabung des Testträgers wird vereinfacht, weil es leichter ist, die Blutprobe zielgenau auf das Testfeld aufzutragen.

Weiterhin ermöglicht es die Erfindung, Testfelder mit einer sehr kleinen Oberfläche (weniger als 50 mm², bevorzugt weniger als 30 mm²) zu verwenden. Dies ist von besonderer Bedeutung, wenn gemäß einer bevorzugten Ausführungsform das Testfeld als Testschichtpaket aus mehreren lose aufeinander aufliegenden Testschichten ausgebildet ist. Dies ist vielfach vorteilhaft, weil auf diese Weise Testschichten mit sehr unterschiedlichen Strukturen miteinander kombiniert werden können. So haben beispielsweise Faserverbundstrukturen (Gewebe oder Vliese) feinporige Kunststoffschichten ("Membranen") und Partikelverbundstrukturen (z.B. EP-A-0013156) jeweils spezielle Eigenschaften, die für bestimmte Einsatzzwecke vorteilhaft sind. Ein Testschichtpaket aus mehreren lose aufeinander aufliegenden Schichten hat in der Regel jedoch einen höheren Flüssigkeitsbedarf je Flächeneinheit, so daß die Möglichkeit, mit einer sehr kleinen Oberfläche zu arbeiten, besonders vorteilhaft ist. Dabei hat es sich überraschenderweise als ausreichend erwiesen, wenn die Schichten des Testschichtpaketes nur von den verhältnismäßig langen und schmalen Andruckarmen gehalten werden.

Die Erfindung wird im folgenden anhand eines in den Figuren dargestellten Ausführungsbeispiels näher erläutert, es zeigen:
- Fig. 1: einen erfindungsgemäßen Testträger in perspektivischer Darstellung,
- Fig. 2: eine Aufsicht auf ein Unterteil,
- Fig. 3: eine Aufsicht auf ein Oberteil,
- Fig. 4: eine vergrößerte Aufsicht auf einen erfindungsgemäßen Testträger,
- Fig. 5: einen vergrößerten Querschnitt durch einen erfindungsgemäßen Testträger entlang der Linie VV in Fig. 4,
- Fig. 6: einen teilweisen Querschnitt entlang der Linie VI-VI in Fig. 4.

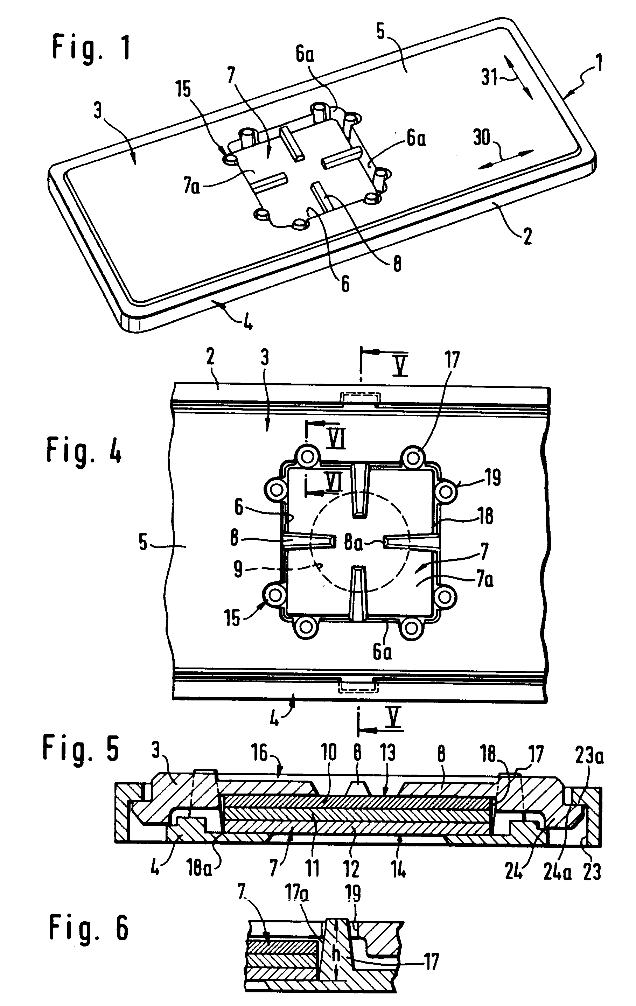

Der in Fig. 1 dargestellte Testträger 1 hat einen flachen, vorzugsweise weniger als 3mm, besonders bevorzugt nur etwa 2mm dicken Rahmen 2, welcher aus einem Oberteil 3 und einem Unterteil 4 besteht. Im Zentrum der oberen Rahmenfläche 5 befindet sich eine erste Testfeldöffnung 6 (Probenauftragsöffnung), durch welche die Oberseite 7a des Testfeldes 7 sichtbar ist. Auf dem Testfeld 7 liegen Andruckarme 8 flach auf, welche vom Rand 6a der Testfeldöffnung 6 ausgehen.

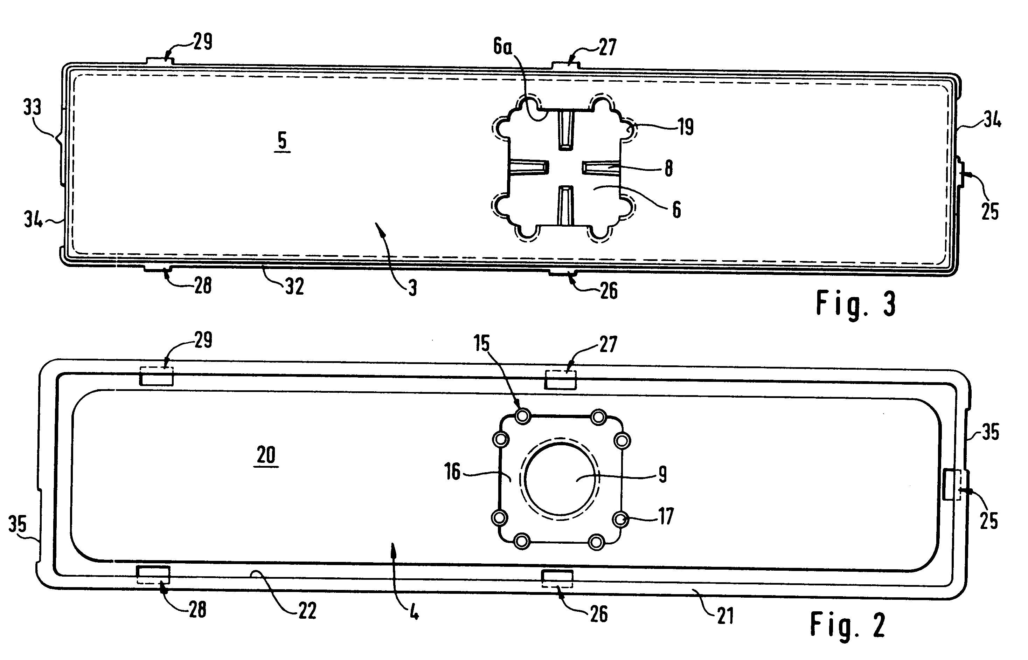

Einzelheiten sind in den Figuren 2 bis 5 dargestellt.

Das Testfeld 7 ist im dargestellten bevorzugten Fall als Testschichtpaket aus drei lose aufeinanderliegenden Testschichten 10,11,12 ausgebildet. Es könnte jedoch auch aus einer einzigen Schicht bzw. einem Schichtverbund bestehen, bei dem die Schichten durch Flüssigbeschichten aufeinander hergestellt und somit vollflächig verbunden sind. Die Schichten erfüllen verschiedene Funktionen im Testablauf. Beispielsweise kann die oberste Testschicht 10 ein Abdecknetz sein, welches die darunterliegenden Schichten schützt und zugleich ein Netzmittel enthält, das die Ausbreitung der Probe fördert. Die Schicht 11 kann zur Abtrennung der Erythrozyten aus der Blutprobe dienen. Geeignet ist beispielsweise ein Glasfaservlies gemäß US-A-4 477 575. Die Schicht 12 kann beispielsweise ein Reagenzfilm sein, welche ein Reagenzsystem enthält, das zu einer für die Analyse charakteristischen farblichen Veränderung auf der Unterseite der Schicht 12 führt. Einzelheiten des Testablaufs sind für die vorliegende Erfindung nicht von Bedeutung. Im Regelfall ist der Testablauf aber so, daß die Probe auf einer Seite des Testschichtpaketes, welche als Probenaufgabeseite 13 bezeichnet werden kann, aufgegeben wird und die Messung bzw. visuelle Auswertung des Nachweissignals auf der anderen Seite des Testschichtpaketes (Nachweisseite 14) durch eine zweite Testfeldöffnung 9 (Meßöffnung) erfolgt.

In dem Unterteil 4 ist eine Aufnahme 16 für das Testfeld 7 vorgesehen. Das Testfeld 7 ist in der Aufnahme von Positioniermitteln 15 umgeben, welche höher als das Testfeld, im dargestellten bevorzugten Fall also höher als das Testschichtpaket 10,11,12 sind.

Die Positioniermittel können von einer die Aufnahme 16 begrenzenden um das Testfeld 7 herumlaufenden geschlossenen Wand gebildet sein. Bevorzugt werden jedoch diskrete, säulenförmige Begrenzungselemente 17 eingesetzt. Die säulenförmigen Begrenzungselemente 17 können verschiedene, beispielsweise dreieckige oder mehreckige Querschnitte aufweisen. Im dargestellten Fall haben die Begrenzungselemente 17 einen kreisrunden Querschnitt. Allgemein hat es sich als vorteilhaft erwiesen, wenn die Berührungsflächen, auf der die Begrenzungselemente in Kontakt mit dem Rand 18 des Testfeldes 7 stehen, möglichst klein sind. Bevorzugt ist eine Form der Begrenzungselemente, die einen Linienkontakt mit dem Rand 18 des Testfeldes 7 gewährleistet. Dies ist beispielsweise bei der dargestellten kreisförmigen Querschnittsform der Fall. Alternativ könnte jedoch auch eine mehreckige Form gewählt werden, bei der eine Ecke oder ein schmaler Steg an dem Rand 18 des Testfeldes 7 anliegt.

Durch die Verwendung diskreter Begrenzungselemente 17 wird einerseits eine exakte Positionierung erreicht. Andererseits ist kein ausgedehnter Kapillarspalt vorhanden, durch den Probenflüssigkeit in unerwünschter Weise abgesaugt werden könnte. Es bleibt ein um den Rand des Testfeldes umlaufender Hohlraum frei, welcher einen Überschuß an Probenflüssigkeit aufnehmen kann. Die gesamte Kontaktfläche, auf der die Begrenzungselemente 17 am Rand 18 das Testfeld 7 kontaktieren, sollte aus diesem Grunde vorzugsweise weniger als 25 %, besonders bevorzugt weniger als 10 % der Gesamtlänge des Testfeldrandes 18 ausmachen.

Gemäß einer weiteren bevorzugten Ausführungsform sind die Begrenzungselemente 17 so ausgebildet, daß ihre dem Testfeld 7 zugewandte Seite 17a von unten nach oben schräg von dem Testfeld weg verläuft. Die Abstände der Begrenzungselemente 17 und die Abmessungen des Testfeldes 7 sind dabei so aufeinander abgestimmt, daß die untere Kante 18a des Randes 18 des Testfeldes 7 von den Begrenzungselementen 17 mit sehr geringem Spiel eingefaßt wird. Dadurch wird die für eine präzise Analyse erforderliche exakte Positionierung erreicht und zugleich eine einfache und rationelle maschinelle Montage ermöglicht. Dies gilt insbesondere für den Fall eines Testschichtpaketes 10,11,12, bei dem die unterste Testschicht 12 sehr genau positioniert wird, während die darüberliegenden Testschichten 11 und 10 mit einem gewissen Spiel in die Aufnahme 16 eingelegt sind.

Das Oberteil 3 weist Ausnehmungen 19 auf, in die die Begrenzungselemente 17 eindringen. Dadurch kann eine für die rationelle Montage des Testträgers 1 erforderliche ausreichende Höhe h der Begrenzungselemente 17 (mindestens 20% mehr als die Gesamtdicke des Testschichtpaketes 10,11,12) vorteilhaft mit einer außerordentlich flachen Bauweise des Rahmens 2 verbunden werden.

In Fig. 4 erkennt man besonders deutlich, daß das Testfeld 7 kleiner als die Probenauftragsöffnung 6 ist, so daß der Rand 18 des Testfeldes 7 abgesehen vom Querschnitt der Andruckarme 8 auf seiner gesamten Länge sichtbar ist. Die Ausnehmungen 19 werden durch Ausbuchtungen in dem Rand 6a der Blutauftragsöffnung 6 gebildet.

Die Andruckarme 8 ragen relativ weit in Richtung auf die Mitte des Testfeldes 7 in die Öffnung 6 hinein. Ihre Länge sollte mindestens etwa 20 %, bevorzugt mindestens 30 % derjenigen Dimension der Testfeldöffnung 6 betragen, in der sich der jeweilige Arm 8 erstreckt. Ihr in die Testfeldöffnung 6 hineinragendes Ende 8a liegt oberhalb der in Fig. 4 gestrichelt angedeuteten unteren Testfeldöffnung 9, durch die die Messung erfolgt.

Zur Verbindung des Oberteils 3 mit dem Unterteil 4 dienen vorzugsweise klebstofffreie diskrete Verbindungselemente 25,26,27,28,29 welche bevorzugt so ausgebildet sind, daß sie jeweils formschlüssig aneinander anliegende, die Relativbewegung senkrecht zur Ebene des Testschichtpaketes 7 begrenzende Begrenzungselemente 23a,24a (Fig. 5) aufweisen. Im dargestellten Fall hat das Oberteil 3 einen Vorsprung 24, dessen obere Fläche eines der Begrenzungselemente bildet. Das Unterteil 4 hat Ausnehmungen 23, welche zum Zentrum des Rahmens 2 hin offen sind, und an denen die Unterteilseitigen Begrenzungselemente 23a ausgebildet sind. Die Vorsprünge 24 greifen in die Ausnehmungen 23 ein. Je ein Vorsprung 24 und eine Ausnehmung 23 bilden ein Verbindungselement 25 bis 29. Abweichende Gestaltungen der Verbindungselemente sind möglich, jedoch ist es vorteilhaft, wenn sie so ausgebildet sind, daß sie einerseits die Relativbewegung zwischen Oberteil und Unterteil in Richtung senkrecht zu den Testschichten 10 bis 12 definiert begrenzen, während sie andererseits eine die elastische Deformierung von Oberteil 3 und Unterteil 4 erleichternde geringfügige Relativbewegung beider Teile in Richtung parallel zur Fläche der Testschichten 10 bis 12 zulassen.

Bevorzugt sind die Verbindungselemente 25 bis 29 als Clipsverbindung ausgebildet, wobei die formschlüssig ineinandergreifenden Teile 23,24 bei der Montage elastisch leicht verformt werden, um sie in Eingriff miteinander zu bringen. Die Clipsverbindung muß so gestaltet sein, daß sie sich bei der erfindungsgemäßen elastischen Verformung nicht lösen kann.

Bei dem dargestellten Ausführungsbeispiel hat das Unterteil 4 ein gegenüber seiner Bodenfläche 20 verdicktes Randprofil 21, an dem die Clipsverbindungen ausgebildet sind.

Im Hinblick auf die gewünschte Elastizität von Oberteil und Unterteil ist es vorteilhaft, wenn beide nur mit verhältnismäßig wenigen diskreten klebstofffreien Verbindungselementen bezüglich einer Relativbewegung senkrecht zur Ebene des Testfelds fixiert sind und diese verhältnismäßig weit auseinanderliegen.

In der Praxis ist auch wichtig, daß Oberteil und Unterteil in Richtung ihrer in Fig. 1 durch die Pfeile 30 und 31 angedeuteten Flächendimensionen exakt zueinander positioniert sind. Zu diesem Zweck hat das Oberteil eine schmale umlaufende Paßleiste 32, die an der Innenwand 22 des Randprofils 21 des Unterteils 20 anliegt. Der exakte Sitz wird durch eine Positioniernase 33 erreicht.

Das Oberteil 3 und das Unterteil 4 sind vorzugsweise in Spritzguß aus Polystyrol oder ABS (Acrylnitril-Butadienstyrol) hergestellt. An ihren Rändern sind zweckmäßigerweise zurückspringende Ausnehmungen 34 bzw. 35 vorgesehen, an denen beim Herstellungsvorgang die Gießzapfen anstehen.

## Patentansprüche

1. Testträger für die Analyse von Flüssigkeiten mit einem in einem Rahmen (2) gehaltenen Testfeld (7), wobei der Rahmen (2) ein Oberteil (3) und ein Unterteil (4) aufweist und mindestens das Oberteil (3) eine Testfeldöffnung (6) aufweist, durch die eine Probenflüssigkeit auf die Oberseite (7a) des Testfeldes (7) aufgebracht werden kann,
**dadurch gekennzeichnet**, daß
das Unterteil (4) eine Aufnahme (16) für das Testfeld (7) aufweist,
die Aufnahme (16) in dem Unterteil (4) von Positioniermitteln (15) für das Testfeld umgeben ist, welche höher als das Testfeld sind,
die Testfeldöffnung (6) größer als das Testfeld (7) ist, so daß der Rand (18) des Testfeldes sichtbar ist, und
das Oberteil (3) in die Testfeldöffnung (7) hineinragende, auf der Oberseite (7a) des Testfeldes (7) aufliegende Andruckarme (8) aufweist.

2. Testträger nach Anspruch 1, **dadurch gekennzeichnet**, daß das Testfeld (7) als Testschichtpaket mit mindestens zwei aufeinander aufliegenden, nicht vollflächig miteinander verbundenen Testschichten (10,11,12) ausgebildet ist.

3. Testträger nach einem der vorhergehenden Ansprüche, **dadurch gekennzeichnet**, daß die Positioniermittel eine Mehrzahl von diskreten, säulenförmigen Begrenzungselementen (17) umfassen.

4. Testträger nach Anspruch 2, **dadurch gekennzeichnet**, daß die Begrenzungselemente (17) so ausgebildet sind, daß sie in Linienkontakt zu dem Rand (18) des Testfeldes (7) stehen.

5. Testträger nach einem der Ansprüche 2 oder 3, **dadurch gekennzeichnet**, daß die Begrenzungselemente (17) auf ihrer dem Testfeld (7) zugewandten Seite (17a) von unten nach oben schräg von dem Testfeld weg verlaufen.

6. Testträger nach einem der Ansprüche 2 bis 4, **dadurch gekennzeichnet**, daß das Oberteil (3) Ausnehmungen (19) aufweist, in die die säulenförmigen Begrenzungselemente (17) eindringen.

7. Testträger nach einem der vorhergehenden Ansprüche, **dadurch gekennzeichnet**, daß das Oberteil (3) und das Unterteil (4) mittels mindestens zwei Verbindungselementen (25,26,27) miteinander verbunden sind, welche jeweils formschlüssig aneinander anliegende, die Relativbewegung der Teile (3,4) senkrecht zur Ebene des Testschichtpaketes (7) begrenzende Begrenzungselemente (23a,24a) aufweisen.

8. Testträger nach Anspruch 7, **dadurch gekennzeichnet**, daß die Verbindungselemente (25,26,27) als Clipsverbindung ausgeführt sind.

9. Testträger nach Anspruch 8, **dadurch gekennzeichnet**, daß das Oberteil (3) oder das Unterteil (4) ein gegenüber dem größten Teil seiner Fläche verdicktes Randprofil (21) hat und die Clipsverbindung in das Randprofil (21) eingreift.

## Claims

1. Test carrier for the analysis of fluids with a test field (7) held in a frame (2), in which the frame (2) comprises a top part (3) and a base part (4) and at least the top part (3) comprises a test field opening (6) through which a sample fluid can be applied to the surface (7a) of the test field (7),
**characterised in that**
the base part (4) comprises a seat (16) for the test field (7),
the seat (16) in the base part (4) is surrounded by positioning means (15) for the test field which are higher than the test field,
the test field opening (6) is greater than the test field (7), so that the rim (18) of the test field is visible, and
the top part (3) comprises pressure tongues (8) resting on the surface (7a) of the test field (7) and projecting into the test field opening (7).

2. Test carrier according to claim 1, **characterised in that** the test field (7) is constructed as a test layer package with at least two superimposed test layers (10, 11, 12) not connected to one another over their whole area.

3. Test carrier according to any one of the preceding claims, **characterised in that** the positioning means incorporate a plurality of discrete, pillar-shaped limiting elements (17).

4. Test carrier according to claim 2, **characterised in that** the limiting elements (17) are so constructed that they stand in line contact with the rim (18) of the test field (7).

5. Test carrier according to any one of claims 2 or 3, **characterised in that** the limiting elements (17) on their side (17a) facing the test field (7) run obliquely away from the test field from bottom to top.

6. Test carrier according to any one of claims 2 to 4, **characterised in that** the top part (3) comprises recesses (19) into which the pillar-shaped limiting elements (17) penetrate.

7. Test carrier according to any one of the preceding claims, **characterised in that** the top part (3) and the base part (4) are connected to one another by means of at least two connection elements (25, 26, 27) which comprise respectively limiting elements (23a, 24a) butting positively against one another and limiting the relative movement of the parts (3, 4) perpendicular to the plane of the test layer package (7).

8. Test carrier according to claim 7, **characterised in that** the connection elements (25, 26, 27) are constructed as clip connections.

9. Test carrier according to claim 8, **characterised in that** the top part (3) or the base part (4) has an edge profile (21) thickened compared with the greatest portion of its surface and the clip connection engages with the edge profile (21).

## Revendications

1. Support de test pour l'analyse de liquides, muni d'un champ de test (7) maintenu dans un cadre (2), le cadre (2) présentant une partie supérieure (3) et une partie inférieure (4) et au moins la partie supérieure (3) présentant une ouverture du champ de test (6) à travers laquelle un liquide-échantillon peut être amené sur la face supérieure (7a) du champ de test (7), caractérisé en ce que la partie inférieure (4) présente un logement (16) pour le champ de test (7), en ce que le logement (16) est entouré, dans la partie inférieure (4), par des moyens de positionnement (15) destinés au champ de test et qui sont situés plus haut que celui-ci, en ce que l'ouverture du champ de test (6) est plus grande que le champ de test (7) de sorte que le bord (18) de celui-ci est visible et en ce que la partie supérieure (3) présente des bras de pression (8) qui font saillie dans l'ouverture du champ de test (7) et qui reposent sur la face supérieure (7a) du champ de test (7).

2. Support de test selon la revendication 1, caractérisé en ce que le champ de test (7) est conçu en tant que paquet-test stratifié avec au moins deux couches-test (10, 11, 12) placées l'une sur l'autre et non reliées entre elles sur toute la surface.

3. Support de test selon l'une des revendications précédentes, caractérisé en ce que les moyens de positionnement comprennent une multitude d'éléments de délimitation (17) discrets et en forme de colonne.

4. Support de test selon la revendication 2, caractérisé en ce que les éléments de délimitation (17) sont conçus de façon à se trouver en contact linéaire avec le bord (18) du champ de test (7).

5. Support de test selon l'une des revendications 2 ou 3, caractérisé en ce que les éléments de délimitation (17) partent obliquement du champ de test, du bas vers le haut, sur leur face (17a) tournée vers le champ de test (7).

6. Support de test selon l'une des revendications 2 à 4, caractérisé en ce que la partie supérieure (3) présente des creux (19) dans lesquels pénètrent les éléments de délimitation (17) en forme de colonne.

7. Support de test selon l'une des revendications précédentes, caractérisé en ce que la partie supérieure (3) et la partie inférieure (4) sont reliées entre elles au moyen de deux éléments de raccord (25, 26, 27) qui présentent chacun des éléments de délimitation (23a, 24a) contigus, à engagement positif et limitant le mouvement relatif des parties (3, 4) verticalement par rapport au plan du paquet-test stratifé (7).

8. Support de test selon la revendication 7, caractérisé en ce que les éléments de raccord (25, 26, 27) sont conçus en tant qu'assemblage clips.

9. Support de test selon la revendication 8, caractérisé en ce que la partie supérieure (3) ou la partie inférieure (4) possède un profil marginal (21) épaissi par la plus grande partie de sa surface et en ce que l'assemblage clips s'engrène dans le profil marginal (21).
